# FASCICULE DE BREVET EUROPEEN

(11) **EP 1 699 445 B1**
(45) Date de publication et mention de la délivrance du brevet: **05.01.2011**
(21) Numéro de dépôt: 04805552.9
(22) Date de dépôt: 25.11.2004
(51) Int. Cl.: A61L 31/10

(54) **COMPOSITION DE REVETEMENT A BASE DE COLLAGENE ET DE POLYSACCHARIDES**
BESCHICHTUNGSZUSAMMENSETZUNG AUF KOLLAGEN- UND POLYSACCHARID-BASIS
COLLAGEN AND POLYSACCHARIDE-BASED COATING COMPOSITION

(30) Priorité: 27.11.2003 FR 0313923
(43) Date de publication de la demande: 13.09.2006
(73) Titulaire: Sofradim Production, 01600 Trévoux (FR)
(72) Inventeur: DARNIS, Thierry, 01480 Frans (FR); THERIN, Michel, 69004 Lyon (FR); LEFRANC, Olivier, 60110 Lormaison (FR); JOZEFONVICZ, Jacqueline, 60260 Lamorlaye (FR)
(74) Mandataire: Brédeville, Odile Marie
(86) Numéro de dépôt international: PCT/FR2004/003023
(87) Numéro de publication internationale: WO 2005/053765

(56) Documents cités:
- EP-A- 0 838 219
- WO-A-03/011355
- FR-A- 2 715 309
- US-B1- 6 177 609
- US-B2- 6 391 052
- CHAUBET FREDERIC ET AL: "Sulphated polysaccharides derived from dextran: biomaterials for vascular therapy" POLYM INT; POLYMER INTERNATIONAL 1999 JOHN WILEY & SONS LTD, CHICHESTER, ENGL, vol. 48, no. 4, 1999, pages 313-319, XP002325917
- LOGEART-AVRAMOGLOU D ET AL: "CARBOXYMETHYL BENZYLAMIDE SULFONATE DEXTRANS (CMDBS), A FAMILY OF BIOSPECIFIC POLYMERS ENDOWED WITH NUMEROUS BIOLOGICAL PROPERTIES: A REVIEW" JOURNAL OF BIOMEDICAL MATERIALS RESEARCH, WILEY, NEW YORK, NY, US, vol. 48, no. 4, 12 juillet 1999 (1999-07-12), pages 578-590, XP001154460 ISSN: 0021-9304 cité dans la demande

## Description

La présente invention a trait au domaine des substrats métalliques revêtus, en particulier à usage médical tels que les endoprothèses (encore appelées stents) utilisées dans le traitement des maladies sténosantes.

Pour rendre ces endoprothèses plus efficaces et mieux tolérées, leur surface peut être recouverte de substances d'origine biologique telles que le collagène, l'héparine, la phosphorylcholine ou encore l'acide désoxyribonucléique (ADN), de polymères tels que les silicones, les polyuréthannes ou les polytétrafluoroéthylène expansés, de céramiques ou oxydes de nature variable (carbone, nitrure ou oxyde de titane, carbure de silicium etc...).

Les différentes modifications de surface peuvent dans certains cas améliorer le comportement in vivo des endoprothèses mais ne suffisent pas toujours à prévenir des complications telles que la resténose.

On connaît aujourd'hui des implants métalliques qui sont recouverts d'agents thérapeutiques, rapamicyne et paclitaxel, qui inhibent au moins partiellement le phénomène de resténose lorsque ces agents sont relargués localement. Ces produits sont commercialisés respectivement par les sociétés Cordis et Boston Scientific.

On connaît de WO99/29734 ou de l'article de D. Logeart-Avramoglou et J. Jozefonvicz paru dans J. Biomed. Mater. Res (Appl. Biomater) 48, 578-590, 1999 des dérivés de dextrane fonctionnalisés qui répondent à la formule DMCaBbSucSd dans laquelle:
D représente une chaîne polysaccharidique, constituée par des enchaînements d'unités α-D-glucopyranosiques reliées entre elles par des liaisons α(1-6) ;
MC représente des groupes méthylcarboxylates ;
B représente des groupes carboxymethylbenzylamides ;
Su représente des groupes sulfates ;
S représente des groupes sulfonates et
a, b, c et d représentent le degré de substitution (ds), exprimé par rapport au nombre de fonctions hydroxyles libres dans une unité glucosidique du dextrane, respectivement en groupements MC, B, Su et S, a étant égal à 0 ou ≥ à 0,2, b étant égal à 0 ou ≥ à 0,1, c étant égal à 0 ou ≥ à 0,1 et d étant égal à 0 ou ≥ à 0,15, à condition que lorsque d=0, a et/ou b soient ≠ 0.

On connaît de WO97/08206, WO01/15654 ou de l'article « Biological activities of polysaccharides from marine algae », C. Boisson-Vidal et al., Drugs of the Future 20 (12) :1237-1249, 1995, des polysaccharides sulfatés de bas poids moléculaire extraits d'algues marines présentant des propriétés antithrombotiques et antiprolifératives.

On connaît de WO03/011355 des implants métalliques, dont des endoprothèses pour angioplastie, qui sont recouvertes de dérivés fonctionnalisés du dextrane liés de manière covalente à la surface de l'endoprothèse en vue de conférer à cette dernière des propriétés biologiques d'intérêt de manière permanente en fixant définitivement sur l'endoprothèse les agents ou composés actifs retenus.

Toutefois, dans certains cas, et notamment lorsque l'on recherche un effet à distance de la surface métallique sur toute la longueur du segment artériel stenté, il est nécessaire que l'endoprothèse ne soit pas recouverte de façon définitive afin d'avoir une diffusion progressive des agents ou composés actifs retenus dans le vaisseau au contact. Il est également particulièrement important que l'endoprothèse soit recouverte de façon uniforme et homogène sur toute sa surface afin d'avoir la meilleure diffusion possible des agents actifs.

Enfin, en particulier lorsque l'endoprothèse, ou stent, est destinée à être déployée ou expansée dans une artère par exemple, il est important que le revêtement ne se déchire pas et ne déforme pas non plus l'endoprothèse lors de cette expansion.

On connaît de WO98/34656 des compositions à base de collagène et d'un dérivé de dextrane pour réaliser des barrières biodégradables utilisées dans la prévention des adhérences post-opératoires.

Il est connu de US 6, 391, 052 des endoprothèses métalliques recouvertes d'un revêtement de collagène pouvant comprendre des glycosaminoglycanes.

Le but de la présente invention est de conférer à un substrat métallique pouvant être utilisé comme endoprothèse, par exemple un stent, des propriétés biologiques d'intérêt, compte tenu de l'application ou de l'indication de ladite prothèse, et ce de manière intégrée de façon temporaire au substrat, c'est à dire sans modifier les propriétés mécaniques intrinsèques dudit substrat d'une part, et en fixant temporairement et de manière uniforme et homogène sur ce substrat les agents ou composés actifs retenus pour exhiber certaines ou la totalité des propriétés biologiques précitées par diffusion progressive ou par action directe de ces agents ou composés actifs.

Un premier objet de la présente invention concerne un substrat métallique, caractérisé en ce qu'il est revêtu d'une composition de revêtement comprenant :
- au moins un composé polysaccharidique,
- au moins un collagène, et
- au moins un mélange de glycérol et de polyéthylène glycol.

Un deuxième objet de la présente invention concerne un procédé de revêtement d'au moins une partie de la surface d'un substrat métallique, caractérisé en ce qu'il comprend les étapes suivantes:
- a°) on prépare une composition de revêtement comprenant i°) au moins un composé polysaccharidique, ii°) au moins un collagène, et iii°) au moins un mélange de glycérol et de polyéthylène glycol,
- b°) on immerge la partie de la surface du substrat à revêtir dans la composition de revêtement obtenue en a°),
- c°) on retire ledit substrat de la composition de revêtement et on laisse sécher.

Un autre objet de l'invention est un substrat métallique revêtu, susceptible d'être obtenu selon le procédé décrit ci-dessus.

Un autre objet de la présente invention est un objet à usage médical ou chirurgical, du type implant, par exemple une endoprothèse vasculaire (dite "stent") pour angioplastie coronarienne transluminale percutanée, comprenant un substrat métallique dont au moins une partie de la surface est revêtue par une composition de revêtement telle que décrite ci-dessus.

Un autre objet de l'invention est une endoprothèse comprenant un substrat métallique revêtu d'une composition de revêtement, tel que décrit ci-dessus.

Ainsi l'invention permet d'obtenir un objet à usage médical ou chirurgical, du type implant, par exemple une endoprothèse vasculaire (dite "stent") pour angioplastie coronarienne transluminale percutanée, qui conserve toutes ses propriétés mécaniques, qui ne déclenche pas de réponse biologique défavorable chez le sujet receveur et évite la resténose.

On entend par propriétés biologiques intrinsèques les activités biologiques précitées et notamment des activités anti-complémentaires et une activité modulatrice de la prolifération des cellules endothéliales et musculaires lisses vasculaires ou encore des propriétés anticoagulantes ou une activité antibactérienne.

La présence des composés polysaccharidiques selon l'invention à la surface de l'endoprothèse permet de développer sur la surface et autour de cette dernière des interactions spécifiques avec le milieu biologique dans lequel elle est implantée ; on observe notamment une inhibition de la prolifération des cellules musculaires lisses humaines et une stimulation de la prolifération des cellules endothéliales au contact de l'endoprothèse, processus qui favorise l'intégration de l'endoprothèse dans le milieu biologique.

En outre, en fonction de leurs degrés de substitution en différents groupements fonctionnalisés, les composés polysaccharidiques décrits ci-dessus peuvent présenter des activités anti-complémentaires et donc anti-inflammatoires, des activités antibactériennes ou encore des propriétés anticoagulantes et antithrombotiques. En outre, ces dérivés peuvent être utilisés comme substituts de plasma sanguin.

Grâce à l'association spécifique selon l'invention dans la composition de revêtement d'un composé polysaccharidique, d'un collagène et d'un mélange de glycérol et de polyéthylène glycol, il est possible d'obtenir une quantité maximale de produit actif à la surface du substrat métallique, cette quantité maximale étant répartie de façon uniforme et homogène à la surface du substrat. Grâce à la composition de revêtement selon l'invention et à l'homogénéité de cette composition, les produits actifs sont libérés progressivement. Cela permet le contrôle de la resténose au niveau de toute la surface de l'endoprothèse par exemple et évite certaines complications ultérieures dues à la mise en place de l'endoprothèse dans le corps du patient.

Les compositions de revêtement de l'invention restent homogènes sur le substrat métallique même lorsque celui-ci est expansé, comme c'est le cas par exemple des stents. Même après expansion du substrat métallique, en particulier du stent, la composition de revêtement selon l'invention ne se déchire pas et ne déforme pas le substrat métallique, en particulier le stent. Il est ainsi possible d'obtenir des stents revêtus particulièrement performants dans le temps.

Les objets ou endoprothèses selon l'invention présentent également comme caractéristiques le fait qu'ils n'entraînent pas la diffusion dans l'organisme de produits toxiques. Ils s'avèrent également non cytotoxiques et non hémolytiques.

Un substrat métallique selon l'invention est un support, dont la surface est destinée à recevoir le revêtement selon l'invention, réalisé en métal ou dont la surface est revêtue d'un métal ou d'un alliage comme les aciers inoxydables, les alliages à base de chrome et de cobalt ou même les superalliages.

Par métal selon l'invention, on entend tout matériau constitué d'un corps simple bon conducteur de la chaleur et de l'électricité, ayant un grand pouvoir réflecteur à l'état poli, et donnant des oxydes qui réagissent avec l'eau pour donner des bases comme le fer, le cobalt, le chrome.

Par alliage, on entend tout produit métallique homogène obtenu par l'association de plusieurs métaux avec une nette prédominance de l'un d'entre eux, en vue de conférer à ce dernier des caractéristiques déterminées.

De préférence, le substrat métallique de l'invention est un alliage, par exemple un acier inoxydable, ou un superalliage, par exemple le phynox.

La composition de revêtement du substrat selon l'invention comprend au moins un composé polysaccharidique.

Par composé polysaccharidique, on entend, au sens de la présente demande, tout polymère, naturel ou synthétique, comprenant une chaîne polymère constituée d'une multiplicité de motifs osidiques encore appelée chaîne polyosidique, ladite chaîne étant substituée ou non.

De préférence, les composés polysaccharidiques de l'invention comprennent au moins un groupe choisi parmi les méthylcarboxylates, les carboxyméthylbenzylamides, les sulfonates, dont les carboxyméthylsulfonates, les sulfates et leurs mélanges. Dans ce cas, au moins une fonction hydroxyle libre d'au moins un motif osidique est substituée par un groupe choisi parmi les méthylcarboxylates, les carboxyméthylbenzylamides, les sulfonates, dont les carboxyméthylsulfonates, les sulfates et leurs mélanges.

Outre des groupes méthylcarboxylates, carboxyméthylbenzylamides, sulfonates, sulfates ou leurs mélanges, les composés polysaccharidiques convenant à l'invention peuvent comprendre au moins un groupe choisi parmi les chaînes comportant des fonctions hydroxyles, des fonctions amide, des groupes benzyles et/ou leurs mélanges.

De préférence, la chaîne polyosidique du composé polysaccharidique est celle d'un polysaccharide choisi dans le groupe constitué par l'amidon, le glycogène, les celluloses, les dextranes, les poly-α-1,3-glucanes, les pullulanes, la chitine, les arabanes, les xylanes, les fucanes, les pectines, les chondroitines sulfates, les mucopolysaccharides et leurs dérivés.

De préférence encore, la chaîne polyosidique du composé polysaccharidique est celle d'un polysaccharide choisi dans le groupe constitué par l'amidon, le glycogène, les celluloses, les dextranes, les poly-α-1,3-glucanes, les pullulanes, la chitine, les arabanes, les xylanes, les fucanes, les pectines, les mucopolysaccharides, et leurs dérivés.

De préférence encore, la chaîne polyosidique du composé polysaccharidique est celle d'un polysaccharide choisi dans le groupe constitué par les dextranes, les fucanes, et leurs dérivés.

De préférence, la chaîne polyosidique du composé polysaccharidique selon l'invention est celle d'un dextrane. De préférence, le dextrane est un polysaccharide constitué d'un enchaînement d'unités α-D-glucopyranosiques reliées entre elles par des liaisons α(1=6).

De préférence, le composé polysaccharidique est un dextrane ayant une masse molaire moyenne comprise entre 10000 et 1000000 g/mol, ayant par exemple une masse molaire égale à 40000, 70000 ou 460000 g/mol.

De préférence, le composé polysaccharidique de la composition de revêtement du substrat de l'invention est choisi parmi les composés de formule générale DMCₐB_{b}Su_{c}S_{d} dans laquelle :
D représente une chaîne polysaccharidique, constituée par des enchaînements d'unités α-D-glucopyranosiques reliées entre elles par des liaisons α(1-6),
MC représente des groupes méthylcarboxylates,
B représente des groupes carboxyméthylbenzylamides,
Su représente des groupes sulfates;
S représente des groupes sulfonates, et
a, b, c et d représentent le degré de substitution (ds), exprimé par rapport au nombre de fonctions hydroxyles libres dans une unité glucosidique de la chaîne polysaccharidique D (ou dextrane), respectivement en groupements MC, B, Su et S, a étant égal à 0 ou ≥à 0,2, b étant égal à 0 ou ≥à 0,1, c étant égal à 0 ou ≥à 0,1 et d étant égal à 0 ou ≤à 0,15, à condition.que lorsque d=0, a et/ou b soient ≠0.

Dans un autre mode de réalisation de l'invention, le composé polysaccharidique est choisi parmi les dérivés de dextrane, possédant un poids moléculaire supérieur à environ 5000 g/mol, constitués de manière statistique de motifs D, MC, B, Su et S, les motifs D étant des motifs osidiques de dextranes, les motifs MC, B Su et S étant tels que définis ci-dessus.

De préférence encore, le composé polysaccharidique est choisi dans le groupe constitué par :
- les composés polysaccharidiques répondant à la formule générale DMCₐB_{b}Su_{c}S_{d} décrite ci-dessus dans laquelle a ≥0,7 ; 0,15 ≤b ≤0,3 ; 0 ≤c ≤ 0,15 et d = 0 ou ≤0,1, et dont la masse molaire moyenne en poids est comprise entre 5 000 et 200 000 g/mol,
- les composés polysaccharidiques répondant à la formule générale DMCₐB_{b}Su_{c}S_{d} décrite ci-dessus dans laquelle 0,4 ≤a ≤0,8 ; 0,3 ≤b ≤0,8 ; 0,1 ≤c ≤0,9 et d = 0 ou ≤0,1, et dont la masse molaire moyenne en poids est comprise entre 5 000 et 200 000 g/mol,
- les composés polysaccharidiques répondant à la formule générale DMCₐB_{b}Su_{c}S_{d} décrite ci-dessus dans laquelle a ≥0,5 ; 0,3 ≤b ≤0,5 ; c = 0 ou ≤ 0,1 et d = 0 ou ≤0,1, et dont la masse molaire moyenne en poids est comprise entre 5 000 et 200 000 g/mol,
- et leurs mélanges.

De tels composés polysaccharidiques, ainsi que leur procédé de préparation, sont décrits dans WO99/29734.

Dans une forme de réalisation de l'invention, le composé polysaccharidique est naturellement porteur de groupes sulfonates et/ou sulfates. De préférence, un tel composé polysaccharidique est choisi parmi l'héparine, les fucanes et leurs mélanges. De préférence encore, un tel composé polysaccharidique est un fucane ou un des dérivés du fucane.

De préférence, le composé polysaccharidique est présent dans la composition de revêtement à une teneur allant de 0,01 % à 20 % en poids, de préférence encore, de 0,1% à 10%, en poids, par rapport au poids total de la composition de revêtement.

La composition de revêtement du substrat selon l'invention comprend également au moins un collagène.

Le collagène utilisable selon la présente invention est de préférence choisi parmi le collagène d'origine animale, le collagène d'origine humaine, comme par exemple le collagène humain placentaire, le collagène obtenu par des moyens de recombinaison génétique et leurs mélanges.

Dans une forme de réalisation de l'invention, le collagène utilisé est du collagène d'origine animale. Le collagène d'origine animale est de préférence du collagène bovin ou porcin de type I.

Le collagène d'origine humaine ou obtenu par recombinaison génétique est de préférence du collagène de type I, de type III, de type IV ou leurs mélanges.

Dans une forme de réalisation de l'invention, on utilise du collagène natif, solubilisé à pH acide ou après traitement par digestion à la pepsine.

Selon un autre mode de réalisation de l'invention, le collagène est modifié par coupure oxydative.

On peut utiliser à cet effet de l'acide périodique ou l'un de ses sels selon la technique décrite par Tardy et coll. (1986) dans la demande FR 2 601 371-A1. Selon la technique décrite dans cette demande, on soumet une solution acide de collagène à l'action de l'acide périodique ou l'un de ses sels par mélange avec une solution de cet acide ou sel à une concentration molaire comprise entre 1 et 10⁻⁵ ; de préférence entre 5x10⁻³M et 10⁻¹M, à une température voisine de la température ambiante, pendant une durée pouvant aller de 10 minutes à 72 heures. Toujours selon cette technique, on utilise une solution acide de collagène dont la concentration est comprise entre 5 et 50 g/l. La concentration en collagène est de préférence 30 g/l. Ce traitement provoque des coupures dans certains constituants du collagène qui sont l'hydroxylysine et les sucres et crée ainsi des sites réactifs sans en provoquer la réticulation. Le collagène oxydé ainsi préparé en solution est chauffé à une température de 37°C ou légèrement supérieure. Il en résulte que la structure hélicoïdale du collagène est dénaturée, au moins partiellement.

Dans une variante de l'invention, on peut supprimer l'étape d'oxydation et utiliser directement la solution de collagène non oxydé initiale.

De préférence, le collagène utilisé est du collagène de type I, extrait de dermes animaux, par solubilisation à pH acide ou par digestion à la pepsine et purifié par précipitations salines; il est utilisé soit après oxydation par l'acide périodique, soit à l'état natif non oxydé.

De préférence, le collagène est présent dans la composition de revêtement à une teneur allant de 0,01 à 10% en poids, de préférence de 0, 1 % à 5% en poids, par rapport au poids total de la composition de revêtement.

La composition de revêtement du substrat selon l'invention comprend également au moins un mélange de glycérol et de polyéthylène glycol.

De préférence, le glycérol est présent dans la composition de revêtement à une teneur allant de 0,1% à 5% en volume, de préférence encore de 0,3% à 3% en volume, par rapport au volume total de la composition de revêtement.

De préférence, le polyéthylène glycol est est présent dans la composition de revêtement à une teneur allant de 0,1% à 5% en poids, de préférence encore de 0,3% à 3% en poids, par rapport au poids total de la composition de revêtement.

Le mélange de glycérol et de polyéthylène glycol permet d'obtenir un revêtement particulièrement uniforme et homogène. Ce mélange améliore les qualités chimiques et physicochimiques du revêtement. Un tel revêtement ne se déchire pas et ne déforme pas le substrat métallique même lorsque ce dernier est expansé. Un tel revêtement permet d'obtenir une quantité maximale de produit actif à la surface du substrat et de libérer ce produit actif de façon progressive au niveau de toute la surface du substrat.

De préférence, selon le procédé selon l'invention, on prépare la composition de revêtement en mélangeant de façon homogène une solution de composé polysaccharidique, une solution de collagène ainsi que le glycérol et le polyéthylène glycol.

De préférence, la solution de composé polysaccharidique comprend, à l'état liquide, entre 7,5 et 15% en poids du composé polysaccharidique en solution aqueuse.

Dans une forme de réalisation de l'invention, la solution de composé polysaccharidique comprend en outre un polymère hydrophile plastifiant, naturel ou synthétique, choisi parmi les polysaccharides non modifiés ou non fonctionnalisés, comme par exemple le dextrane, ou parmi les polysaccharides substitués par des fonctions carboxylates.

Ainsi, de préférence, la composition de revêtement comprend, outre le composé polysaccharidique, le collagène et le mélange de glycérol et de polyéthylène glycol décrits ci-dessus, au moins un polymère hydrophile plastifiant, naturel ou synthétique, choisi parmi les polysaccharides non modifiés ou non fonctionnalisés, comme par exemple le dextrane, les polysaccharides substitués par des fonctions carboxylates, et leurs mélanges.

Lors du mélange de la solution de composé polysaccharidique et de la solution de collagène, l'association du collagène avec le composé polysaccharidique se fait par interactions électrostatiques fortes de préférence avec les groupes porteurs de charges négatives de ce dernier, comme les groupes méthylcarboxylates, carboxyméthylbenzylamides, sulfates ou/et sulfonates. Ces interactions peuvent être complétées par une réticulation finale du réseau collagénique par tous moyens connus tels que l'addition de glutaraldéhyde, de polyaldéhydes macromoléculaires, ou autres agents chimiques, ou enfin par irradiation avec des sources ionisantes : U.V. ; Cobalt; Faisceau d'électrons.

Le collagène est lié au composé polysaccharidique par formation d'un polyélectrolyte complexe grâce aux charges électriques complémentaires des deux principaux constituants que sont le collagène et le composé polysaccharidique.

De préférence, selon le procédé selon l'invention, on immerge la partie de la surface du substrat métallique à revêtir dans la composition de revêtement comprenant le complexe de collagène et de composé polysaccharidique, et le mélange de glycérol et de polyéthylène glycol. Selon le procédé de l'invention, le composé polysaccharidique mis en oeuvre conserve ses propriétés biologiques intrinsèques après revêtement du substrat malgré les liaisons électrostatiques avec le collagène.

De préférence, on immerge la partie de la surface du substrat métallique à revêtir dans la composition de revêtement afin de réaliser une enduction. Pour ce faire, la composition de revêtement est de préférence liquide. Elle peut être diluée si nécessaire. De préférence, cette composition présente une viscosité allant de 25 10⁻³ à 0,1 Pa.s mesurée sur un rhéomètre Rheovisco L2 (Rheo, Shannon Irlande) permettant la mesure de la viscosité dynamique par la résistance à la rotation d'un rotor dans la composition. L'enduction a lieu de préférence à une température comprise entre 30°C et 50°C, à un pH compris entre 5 et 8, durant un temps compris entre 2 secondes et 5 min. Pour réaliser une enduction optimale, l'immersion de la surface du substrat dans la composition de revêtement est de préférence réitérée à un nombre de reprises allant de 1 à 10.

Selon le procédé de l'invention, on retire ensuite le substrat de la composition de revêtement et on laisse sécher la partie revêtue de sa surface. De préférence, le séchage se fait sous rotation constante afin d'obtenir une couche ou un film de revêtement homogène sur toute la surface revêtue dudit substrat métallique. De préférence la surface revêtue est mise à sécher sous hotte à une température comprise entre 10 °C et 35°C et durant une durée comprise entre 6 et 36 heures.

Dans une forme préférée de réalisation de l'invention, le procédé de l'invention comprend en outre une étape de stérilisation du substrat revêtu. De préférence, cette stérilisation se fait par traitement au gaz d'oxyde d'éthylène.

De préférence, l'épaisseur de la couche ou du film de revêtement, mesurée par microscopie électronique à balayage, va de 20 nm à 100 microns, et de préférence encore de 1 micron à 100 microns.

Selon le procédé de l'invention, et en particulier du fait du mélange spécifique du glycérol et du polyéthylène glycol dans la composition de revêtement, on obtient un revêtement particulièrement uniforme et homogène qui permet la libération progressive des actifs au niveau de toute la surface du substrat métallique.

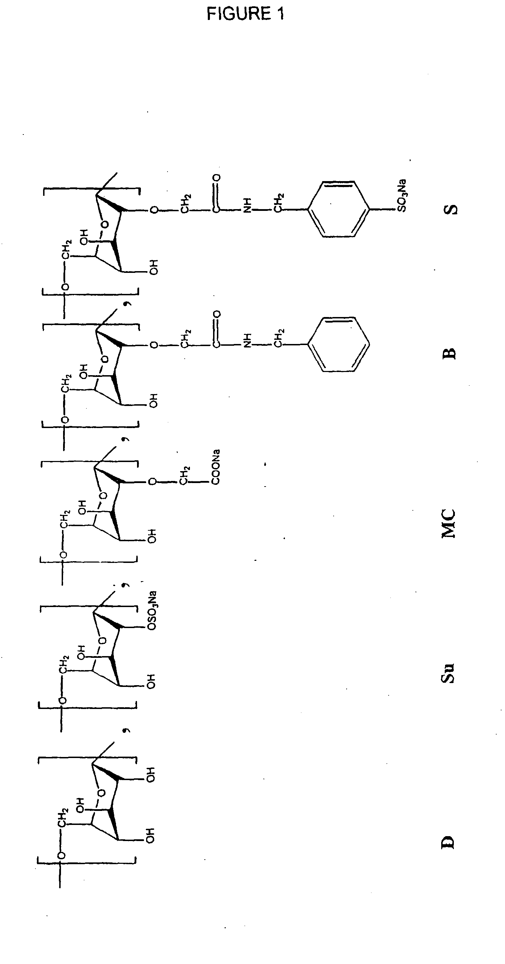

- La figure 1 représente schématiquement la structure d'un dérivé fonctionnalisé de dextrane substitué par les différents groupements chimiques MC, B, Su et S fixés sur les unités glucosidiques D ; à titre d'exemple, la position du substituant sur les différents carbones des unités glucosidiques est présentée sur le carbone 2.

Les exemples suivants illustrent l'invention.

### EXEMPLE 1 : préparation du collagène

Le collagène de cet exemple est réalisé comme décrit dans le brevet Tardy et coll. : FR 8610160.

Le collagène utilisé est du collagène bovin de type I en solution aqueuse à 3% (m/V), extrait de dermes animaux, par solubilisation à pH acide ou par digestion à la pepsine et purifié par précipitations salines selon des techniques déjà décrites. Ce collagène oxydé est composé d'un enchevêtrement de trois chaînes aminées α de 100 000 g/mol chacune.

On utilise de préférence des fibres sèches de collagène, obtenues par précipitation d'une solution acide de collagène par addition de NaCl, puis par lavages et séchage du précipité obtenu par des solutions aqueuses d'acétone de concentration croissante de 80% à 100%.

Une solution de collagène à 30 g/l est préparée par dissolution en HCl 0,01 N. Son volume est de 49 litres. Elle est additionnée d'acide périodique à une concentration finale de 8 mM soit 1,83 g/l. On réalise l'oxydation à température ambiante voisine de 22°C, pendant 3 heures à l'abri de la lumière. Puis la solution est additionnée d'un volume égal d'une solution de chlorure de sodium pour obtenir une concentration finale de 41 g/l de NaCl. Après 30 minutes d'attente, le précipité est récolté par décantation à travers un tamis de toile de porosité voisine de 100 microns, puis lavé 4 fois avec une solution de NaCl 41 g/l en HCl 0,01N. On obtient 19 Kg de précipité de collagène salin acide. Ces lavages permettent l'élimination de toutes traces d'acide périodique ou dérivés iodés formés pendant l'oxydation du collagène. Ensuite plusieurs lavages en solution aqueuse d'acétone à 80% permettent la concentration du précipité de collagène et l'élimination des sels présents. Un lavage final en acétone 100% permet de préparer 3,6 Kg d'un précipité très dense de collagène oxydé acide, non réticulé, ne contenant aucune trace de produit chimique indésirable.

### EXEMPLE 2: Préparation des compositions de revêtement des endoprothèses vasculaires

### 1) Support et solutions utilisés

Le support utilisé consiste en une plaque de polychlorure de vinyle.

La solution de revêtement est une solution de collagène de type I ou I+III, tel que préparé à l'Exemple 1 de la présente demande, à 2,4% en poids, de glycérol à 2% en volume et de polyéthylène glycol (PEG) à 2% en poids mélangés à un composé polysaccharidique.

Le composé polysaccharidique utilisé est un dérivé fonctionnalisé du dextrane, de masse molaire moyenne en poids 70 000 g/mol, de formule DMC_{0,6}B_{0,45}Su_{0,6}, correspondant à la formule générale DMCₐB_{b}Su_{c}S_{d} telle que précédemment décrite dans la présente demande.

Son protocole de préparation est tel que décrit dans le brevet européen publié sous le numéro 0146455. Ce dérivé fonctionnalisé du dextrane inhibe la prolifération des cellules musculaires lisses mais inhibe aussi l'activation du système du complément (test de CH50 réalisé) et stimule la prolifération des cellules endothéliales vasculaires.

Ses activités spécifiques anticoagulantes et anticomplémentaires sont respectivement de 3,5 Ul/mg et 0,13 µg/mL par comparaison à l'héparine dont l'activité anticoagulante est de 173 Ul/mg et l'activité anticomplémentaire de 44,5 µg/mL.

### 2) Protocole

Une solution composée de collagène, de PEG et de glycérol respectivement à 2,4% en poids, 2% en poids et 2% en volume est préparée à 37°C. Le pH de cette solution est fixé à 7 par ajout de NaOH.

Une solution de DMC_{0,6}B_{0,45}Su_{0,6}, tel que décrit au point 1°) ci-dessus, à 10% (m/V), est préparée dans la solution de collagène-PEG-glycérol telle que décrite ci-dessus. Le pH est contrôlé et éventuellement réajusté à 7.

La composition de revêtement ainsi obtenue est versée sur un film de PVC à raison de 0,133 g/cm² puis laissée à sécher sous hotte durant 16 heures.

### EXEMPLE 3: Préparation de stents en aciers inoxydables selon la présente invention, nettoyage, revêtement et séchage

### 1) Support et solutions utilisés

Le support utilisé consiste en un stent en acier inoxydable fourni par la société SOFRADIM sous la dénomination commerciale « MedXflexy® » et répondant aux normes NF EN 12006-3 (Implants chirurgicaux non actifs. Exigences particulières relatives aux implants cardiaques et vasculaires. Partie 3 : Prothèses endovasculaires (stents)) et ISO 25539-1 (Cardiovascular implants - Endovascular devices - Part 1 : endovascular prostheses.)

Les compositions de revêtement utilisées sont telles que décrites dans l'exemple 2 mais diluées avec 80%vol. d'eau distillée. Cette dilution permet d'éviter la formation de ponts de collagène entre les mailles du stent et ainsi de ne pas déformer le stent lors de sa mise en place sur son ballonnet.

### 2) Protocole

Une composition de collagène-dérivé du dextrane-PEG-Glycérol respectivement à 0,48% en poids; 2% en poids; 0,4% en poids et 0,4% en volume est préparée telle que décrite au point 1°) ci-dessus.

Un stent est immergé verticalement 10 secondes dans la composition ainsi préparée puis extrait et laissé à sécher sous rotation durant 1 minute. L'opération est renouvelée 6 fois afin d'assurer un revêtement suffisant du stent.

Le stent enduit est alors laissé à sécher définitivement en position verticale durant 16 heures sous hotte à flux laminaire.

Le stent enduit est alors serti mécaniquement sur son ballonnet de dilatation de type PTA (percutaneous transluminal angioplasty), PTCA (percutaneous transluminal coronary angioplasty) ou PTRA (percutaneous transluminal renal angioplasty) puis stérilisé en présence d'oxyde d'éthylène ou par rayonnement afin de faciliter la réticulation du film à base de collagène.

### EXEMPLE 4 : Préparation de stents en acier inoxydable sertis sur leur ballonnet selon la présente invention, revêtement et séchage

### 1) Support et solutions utilisés

Le support utilisé consiste en un stent en acier inoxydable monté sur son ballonnet fourni par la société SOFRADIM sous la dénomination commerciale « MedXFlexy® » et répondant aux normes NF EN 12006-3 (Implants chirurgicaux non actifs. Exigences particulières relatives aux implants cardiaques et vasculaires. Partie 3 : Prothèses endovasculaires (stents)) et ISO 25539-1 (Cardiovascular implants - Endovascular devices - Part 1: endovascular prostheses).

Les compositions de revêtement utilisées sont telles que décrites dans l'exemple 2 mais diluées avec 25%vol. d'eau distillée. Cette dilution permet d'éviter la formation de ponts de collagène entre les mailles du stent empêchant ainsi le déchirement du collagène lors de l'expansion du stent.

### 2) Protocole

Une composition de collagène-dérivé du dextrane-PEG-Glycérol respectivement à 1,8% en poids; 7,5% en poids; 1,5% en poids et 1,5% en volume est préparée telle que décrite au point 1°) ci-dessus.

Un stent serti est immergé verticalement 10 secondes dans la composition ainsi préparée puis extrait et laissé à sécher sous rotation durant 1 minute. L'opération est renouvelée 3 fois afin d'assurer un revêtement suffisant du stent. Le bout du ballonnet (extrémité distale) est débarrassé de la composition en excès par essuyage.

Le stent serti enduit est alors laissé à sécher définitivement en position semi-verticale durant 16 heures sous hotte à flux laminaire puis stérilisé par traitement au gaz d'oxyde d'éthylène.

Le revêtement obtenu est particulièrement uniforme et homogène, en particulier grâce au mélange de glycérol et de polyéthylène glycol.

### EXEMPLE 5 : Analyse de la surface des stents enduits

### 1) Description des appareils

Afin de vérifier l'homogénéité du revêtement à la surface des stents, ceux-ci sont observés et leurs surfaces caractérisées chimiquement.

Les méthodes d'analyse de la surface employées sont l'XPS (Spectroscopie de Photoelectron X) et le MEB (Microscope électronique à balayage).

### 2) Protocole

L'appareil XPS employé est un ESCA S-Probe de Surface Science Instruments (SSI) avec source Al monochromatisée sous ultravide. Cet appareil analyse plusieurs surfaces de 0,3x1,2 mm² sur une profondeur de 10 nm.

Le MEB utilisé est un Leica S-440. Les échantillons sont métallisés par dépôt d'Or-Palladium sur leurs surfaces.

### 3) Résultats

L'analyse XPS des stents tels que préparés et enduits à l'exemple 3, puis stérilisés à l'oxyde d'éthylène, a montré un revêtement homogène sur toute la surface des stents. L'indexation des pics est attribuée aux éléments du collagène et du dérivé du dextrane. Le substrat métallique n'est pas atteint par le faisceau d'électron, ce qui montre que la couche de revêtement est supérieure à 10 nm.

L'analyse MEB a montré, selon les deux modes d'enduction, un revêtement homogène sur la surface et la tranche des fils du stent. L'épaisseur du revêtement a été évaluée à environ 1 µm en moyenne à la surface des fils du stent.

### EXEMPLE 6 : Test de cytotoxicité du revêtement par essai de diffusion des extraits

### 1) Test, cellules témoins et échantillons utilisés

Le test employé est un test de mesure indirecte basée sur la toxicité des produits diffusés ou relargués par le film de revêtement. Ce test suit la norme internationale ISO 10993-5, concernant l'évaluation biologique des dispositifs médicaux.

Les supports utilisés sont des plaques de culture cellulaire 24 puits commercialisées par la société Corning Costar.

Le composé polysaccharidique est le dérivé de dextrane décrit dans l'exemple 2.

Les cellules employées sont des cellules musculaires lisses immortalisées d'aorte de rats de la lignée BLC#5 en phase de croissance exponentielle ensemencées à 10000 cellules par puits.

Le véhicule d'extraction ainsi que le témoin négatif est un milieu de culture à 10% (VN) en sérum de veau foetal (SVF).

Les échantillons utilisés sont des puits au fond desquels a été coulé un gel composé de collagène-PEG-Glycérol ou un gel composé de collagène-derivé du dextrane-PEG-Glycérol, tel que décrit dans l'exemple 2, ainsi qu'une solution d'extraction contenant 2% (VN) de glycérol.

### 2) Protocole

Les gels sont préparés de manière stérile puis coulés au fond des puits de plaques de culture. Ils sont alors laissés à sécher 24 heures en incubateur air/CO₂. Les puits sont alors remplis de 1,5 mL de milieu de culture à 10% (VN) SVF, et de milieu de culture à 10% (VN) SVF et 2% (VN) glycérol dans le cas d'une plaque vide. Les plaques sont alors placées pendant 72 heures dans un incubateur air/CO₂.

Après 72 heures d'incubation, les extraits sont récupérés et mis au contact des cellules BLC#5 en début de phase de croissance exponentielle.

La densité cellulaire est déterminée au bout de 5 jours d'incubation à l'aide d'un compteur de cellules (coulter counter « Coultronic »). Chaque expérience est effectuée trois fois. Les résultats sont donnés sous la forme d'une moyenne des trois mesures effectuées.

### 3) Résultats

Le tableau I ci-dessous donne le nombre de cellules par puits au bout de 5 jours d'incubation.

**Tableau I : Effet des surfaces par diffusion des extraits sur les cellules musculaires lisses**

| Témoin négatif | Gel de collagène-Glycérol-PEG | Collagène-dérivé du dextrane-glycérol-PEG | Glycérol |
|---|---|---|---|
| 680570 | 277000 | 112640 | 210000 |

Un effet d'inhibition de la prolifération des cellules musculaires lisses a été constaté pour la composition comprenant le composé polysaccharidique et le collagène. Dans ce dernier cas, il semble que le relargage rapide de glycérol soit la cause de cette inhibition.

### EXEMPLE 7 : Propriétés biologiques d'un film de collagène-dérivé du dextrane PEG-glycérol

### 1) Test, supports et cellules utilisés

Le test utilisé est un test de prolifération des cellules directement sur un support enduit.

Les supports et les produits utilisés sont tels que décrits dans l'exemple 6.

Les cellules utilisées sont les cellules endothéliales humaines immortalisées de la lignée EAhy 926.

### 2) Protocole

Les cellules endothéliales sont ensemencées au fond des puits enduits de gel de collagène-PEG-Glycérol et de gel de collagène-dérivé du dextrane-PEG-Glycérol ainsi que dans des puits témoins.

Les cellules sont ensemencées à raison de 10000 cellules par puits. Les cellules sont maintenues 7 jours dans un incubateur air/CO2. Elles sont détachées toutes les 24 heures au moyen de la trypsine-EDTA et leur nombre est déterminé au compteur de cellules.

Les milieux des puits à 10 % (V/V) de SVF sont changés toutes les 48 heures.

### 3) Résultats

Les résultats du test sont donnés dans le tableau II ci-dessous. Ils sont présentés en nombre de cellules par cm².

Une meilleure prolifération est observée sur les films de collagène-PEG-Glycérol et sur les films de collagène-dérivé du dextrane-PEG-Glycérol que sur les puits témoins.

Ces résultats indiquent que la prolifération des cellules endothéliales est favorisée par le support à base de collagène, et que, en outre, la présence du dérivé fonctionnalisé du dextrane stimule cette prolifération.

**Tableau II : Nombre de cellules par cm² en fonction du temps et de la surface**

| | Puits témoins | Collagène-PEG-Glycérol | Collagène-dérivé du dextrane-PEG-Glycérol |
|---|---|---|---|
| T0 | 5000 | 5000 | 5000 |
| J1 | 3500 | 6500 | 7200 |
| J2 | 8000 | 17000 | 15500 |
| J4 | 22500 | Collagène dissous | 58000 |
| J5 | 55000 | Collagène dissous | 76000 |
| J7 | 90000 | Collagène dissous | 98500 |

### EXEMPLE 8 : Propriétés biologiques d'un film de collagène-dérivé du dextrane-PEG-glycérol

### 1) Test, supports et cellules utilisés

Le test utilisé est un test de prolifération des cellules directement sur un support enduit.

Les supports et produits utilisés sont tels que décrits dans l'exemple 6.

Les cellules utilisées sont les cellules musculaires lisses d'aorte de rat de la lignée BLC#5.

### 2) Protocole

Les cellules musculaires lisses sont ensemencées au fond des puits enduits de gel de collagène-PEG-Glycérol et de gel de collagène-dérivé du dextrane-PEG-Glycérol ainsi que dans des puits témoins.

Les cellules sont ensemencées à raison de 10000 cellules par puits. Les cellules sont maintenues 7 jours dans un incubateur air/CO2. Elles sont décrochées toutes les 24 heures grâce à de la trypsine-EDTA et comptées au compteur de cellules.

Les milieux des puits à 10 % de SVF sont changés toutes les 48 heures.

### 3) Résultats

Les résultats du test sont donnés dans le tableau III ci-dessous. Ils sont présentés en nombre de cellules par cm².

Une meilleure prolifération est observée sur les films de collagène-PEG-Glycérol que sur les puits témoins. La présence de dérivé du dextrane dans le gel inhibe très fortement l'adhérence et la prolifération des cellules musculaires lisses à la surface des puits.

**Tableau III : Nombre de cellules par cm² en fonction du temps et de la surface**

| | Puits témoins | Collagène-PEG-Glycérol | Collagène-dérivé du dextrane-PEG-Glycérol |
|---|---|---|---|
| T0 | 5000 | 5000 | 5000 |
| J1 | 13800 | 34500 | 495 |
| J2 | 55500 | 78900 | 555 |
| J4 | 306300 | 645000 | 745 |
| J5 | 476000 | Collagène dissous | 1062 |
| J7 | 747500 | Collagène dissous | 4515 |

### EXEMPLE 9 : résistance de la composition de revêtement

La résistance de la composition de revêtement selon l'invention a été mesurée pour différents taux de glycérol et différents taux de polyéthylène glycol (PEG).

Les supports utilisés sont des stents en acier inoxydable fournis par la société SOFRADIM sous la dénomination commerciale « MedXflexy® » et répondant aux normes NF EN 12006-3.

On a préparé des compositions de revêtement de composition suivante (les quantités sont données en % (m/V) :
- composé polysaccharidique 0,1 %
- collagène 2,4 %
- PEG 2 %
- glycérol X %
- eau qsp 100%
dans laquelle :
- le composé polysaccharidique est celui de l'Exemple 3 de la présente demande
- le collagène est tel que préparé à l'Exemple 1 de la présente demande,
- la valeur de X est donnée dans le tableau IV suivant :

**Tableau IV :**

| Composition | X (Glycérol en % (V/V)) |
|---|---|
| Composition 1 (comparative) | 0 |
| Composition 2 (invention) | 1,5 |
| Composition 3 (invention) | 2 |
| Composition 4 (invention) | 2,4 |
| Composition 5 (invention) | 3 |

Chaque composition i, i allant de 1 à 5, a été utilisée pour revêtir un stent selon le procédé décrit dans l'Exemple 3 de la présente demande. A chaque composition i, correspond ainsi un stent i. Les stents 1 à 5 ont ensuite été expansés, à 4 mm de diamètre, après avoir été immergés dans une solution tampon de phosphate saline (PBS) à 37°C pendant environ 60 secondes.

La résistance de la composition revêtant un stent est observée qualitativement à l'aide d'un microscope optique à transmission. Selon la présente demande, on considère qu'une composition est résistante à l'expansion si le film qu'elle forme sur le stent ne se déchire pas et si ce film ne déforme pas le stent après l'expansion décrite ci-dessus.

Toujours selon la présente demande, on considère qu'un film est étirable s'il subit une déformation élastique ou plastique significative lorsque soumis à des contraintes de déformation sous traction, avec ou sans retour à l'état d'origine après libération des contraintes.

Les résultats de tests d'expansion pour les compositions 1 à 5 revêtant respectivement les stents 1 à 5 sont regroupés dans le tableau V suivant:

**Tableau V :**

| Composition | Etirabilité | Résistance à l'expansion |
|---|---|---|
| Composition 1 (comparative) | Non | Non |
| Composition 2 (invention) | Oui | Non |
| Composition 3 (invention) | Oui | Oui |
| Composition 4 (invention) | Oui | Oui |
| Composition 5 (invention) | Oui | Oui |

Il apparaît clairement dans cette demande que la présence de glycérol dans les compositions de revêtement des substrats métalliques selon l'invention, comme par exemple des stents, améliore l'étirabilité et la résistance à l'expansion de ces compositions.

On a également préparé des compositions de revêtement de composition suivante (les quantités sont données en % (m/V) :
- composé polysaccharidique 0,1 %
- collagène 2,4 %
- PEG Y %
- glycérol 2 %
- eau qsp 100%
dans laquelle :
- le composé polysaccharidique est celui de l'Exemple 3 de la présente demande,
- le collagène est tel que préparé à l'Exemple 1 de la présente demande, la valeur de Y est donnée dans le tableau VI suivant :

**Tableau VI :**

| Composition | Y (PEG en % (m/V)) |
|---|---|
| Composition 6 (comparative) | 0 |
| Composition 7 (invention) | 1,5 |
| Composition 8 (invention) | 2 |
| Composition 9 (invention) | 2,4 |

Chaque composition i, i allant de 6 à 9, a été utilisée pour revêtir un stent selon le procédé décrit dans la l'Exemple 3 de la présente demande. A chaque composition i, correspond ainsi un stent i. Les stents 6 à 9 ont ensuite été expansés, à 4 mm de diamètre, après avoir été immergés dans une solution tampon de phosphate saline (PBS) à 37°C pendant environ 60 secondes.

Les résultats de tests d'expansion des compositions 6 à 9 revêtant respectivement les stents 6 à 9 sont regroupés dans le tableau VII suivant:

**Tableau VII :**

| Composition | Etirabilité | Résistance à l'expansion |
|---|---|---|
| Composition 6 (comparative) | Non | Non |
| Composition 7 (invention) | Oui | Non |
| Composition 8 (invention) | Oui | Oui |
| Composition 9 (invention) | Oui | Oui |

Là aussi, il apparaît clairement de ces résultats que la présence de polyéthylène glycol dans les compositions de revêtement des substrats métalliques selon l'invention, comme par exemple des stents, améliore l'étirabilité et la résistance à l'expansion de ces compositions.

## Revendications

1. Substrat métallique, **caractérisé en ce qu'**il est revêtu d'une composition de revêtement comprenant :
- au moins un composé polysaccharidique,
- au moins un collagène, et
- au moins un mélange de glycérol et de polyéthylène glycol.

2. Substrat selon la revendication 1, **caractérisé en ce que** le composé polysaccharidique comprend une chaîne polymère constituée d'une multiplicité de motifs osidiques encore appelée chaîne polyosidique, ladite chaîne étant substituée ou non.

3. Substrat selon la revendication 1 ou 2, **caractérisé en ce que** le composé polysaccharidique comprend au moins un groupe choisi parmi les méthylcarboxylates, les carboxyméthylbenzylamides, les sulfonates, dont les carboxyméthylsulfonates, les sulfates et leurs mélanges

4. Substrat selon l'une quelconque des revendications précédentes, **caractérisé en ce que** le composé polysaccharidique comprend en outre au moins un groupe choisi parmi les chaînes comportant des fonctions hydroxyles, des fonctions amide, des groupes benzyles et/ou leurs mélanges.

5. Substrat selon l'une quelconque des revendications 2 à 4, **caractérisé en ce que** la chaîne polyosidique du composé polysaccharidique est celle d'un polysaccharide choisi dans le groupe constitué par l'amidon, le glycogène, les celluloses, les dextranes, les poly-α-1,3-glucanes, les pullulanes, la chitine, les arabanes, les xylanes, les fucanes, les pectines, les chondroitines sulfates, les mucopolysaccharides et leurs dérivés.

6. Substrat selon la revendication 5, **caractérisé en ce que** la chaîne polyosidique du composé polysaccharidique est celle d'un dextrane.

7. Substrat selon la revendication 6, **caractérisé en ce que** le composé polysaccharidique est un dextrane ayant une masse molaire moyenne comprise entre 10000 et 1000000 g/mol), ayant par exemple une masse molaire égale à 40000, 70000 ou 460000 g/mol.

8. Substrat selon la revendication 6 ou 7, **caractérisé en ce que** le composé polysaccharidique est choisi parmi les composés de formule générale DMCₐB_{b}Su_{c}S_{d} dans laquelle :
D représente une chaîne polysaccharidique, constituée par des enchaînements d'unités α-D-glucopyranosiques reliées entre elles par des liaisons α(1-6),
MC représente des groupes méthylcarboxylates,
B représente des groupes carboxyméthylbenzylamides,
Su représente des groupes sulfates,
S représente des groupes sulfonates, et
a, b, c et d représentent le degré de substitution (ds), exprimé par rapport au nombre de fonctions hydroxyles libres dans une unité glucosidique de la chaîne polysaccharidique D, respectivement en groupements MC, B, Su et S, a étant égal à 0 ou ≥à 0,2, b étant égal à 0 ou ≥à 0,1, c étant égal à 0 ou ≥à 0,1 et d étant égal à 0 ou ≤à 0,15, à condition que lorsque d=0, a et/ou b soient ≠ 0.

9. Substrat selon la revendication 8, **caractérisé en ce que** le composé polysaccharique est choisi dans le groupe constitué par :
- les composés polysaccharidiques répondant à la formule générale DMCₐB_{b}Su_{c}S_{d} dans laquelle a ≥0,7 ; 0,15 ≤b ≤0,3; 0 ≤c ≤0,15 et d = 0 ou ≤ 0,1, et dont la masse molaire moyenne en poids est comprise entre 5 000 et 200 000 g/mol,
- les composés polysaccharidiques répondant à la formule générale DMCₐB_{b}Su_{c}S_{d} dans laquelle 0,4 ≤a ≤0,8 ; 0,3 ≤b ≤0,8 ; 0,1 ≤c ≤0,9 et d = 0 ou ≤0,1, et dont la masse molaire moyenne en poids est comprise entre 5 000 et 200 000 g/mol,
- les composés polysaccharidiques répondant à la formule générale DMCₐB_{b}Su_{c}S_{d} dans laquelle a ≤0,5 ; 0,3 ≤b ≤0,5 c = 0 ou ≤0,1 et d = 0 ou ≤ 0,1, et dont la masse molaire moyenne en poids est comprise entre 5 000 et 200 000 g/mol,
- et leurs mélanges.

10. Substrat selon la revendication 1, **caractérisé en ce que** le composé polysaccharidique est naturellement porteur de groupes sulfonates et/ou sulfates.

11. Substrat selon la revendication 10, **caractérisé en ce que** le composé polysaccharidique est choisi parmi l'héparine, les fucanes et leurs mélanges.

12. Substrat selon l'une quelconque des revendications précédentes, **caractérisé en ce que** le collagène est choisi parmi le collagène d'origine animale, le collagène d'origine humaine, comme par exemple le collagène humain placentaire, le collagène obtenu par des moyens de recombinaison génétique et leurs mélanges.

13. Substrat selon la revendication 12, **caractérisé en ce que** le collagène est du collagène d'origine animale.

14. Substrat selon la revendication 12, **caractérisé en ce que** le collagène d'origine animale est du collagène bovin ou porcin de type 1.

15. Substrat selon l'une quelconque des revendications 12 à 14, **caractérisé en ce que** le collagène est du collagène natif, solubilisé à pH acide ou après traitement par digestion à la pepsine.

16. Substrat selon l'une quelconque des revendications 12 à 14, **caractérisé en ce que** le collagène est du collagène modifié par coupure oxidative.

17. Substrat selon l'une quelconque des revendications 1 à 13, **caractérisé en ce que** le collagène est du collagène de type I, extrait de dermes animaux, par solubilisation à pH acide ou par digestion à la pepsine et purifié par précipitations salines.

18. Substrat selon l'une quelconque des revendications 1 à 17, **caractérisé en ce que** le composé polysaccharidique est présent dans la composition de revêtement à une teneur allant de 0,01 % à 20 % en poids, de préférence encore, de 0,1% à 10%, en poids, par rapport au poids total de la composition de revêtement

19. Substrat selon l'une quelconque des revendications 1 à 18, **caractérisé en ce que** le collagène est présent dans la composition de revêtement à une teneur allant de 0.01% à 10% en poids, de préférence de 0,1% à 5% en poids, par rapport au poids total de la composition de revêtement.

20. Substrat selon l'une quelconque des revendications 1 à 19, **caractérisé en ce que** le glycérol est présent dans la composition de revêtement à une teneur allant de 0,1% à 5% en volume, de préférence encore de 0,3% à 3% en volume, par rapport au volume total de la composition de revêtement.

21. Substrat selon l'une quelconque des revendications 1 à 20, **caractérisé en ce que** le polyéthylène glycol est présent dans la composition de revêtement à une teneur allant de 0,1% à 5% en poids, de préférence encore de 0,3% à 3% en poids, par rapport au poids total de la composition de revêtement.

22. Procédé de revêtement d'au moins une partie de la surface d'un substrat métallique, **caractérisé en ce qu'**il comprend les étapes suivantes :
- a°) on prépare une composition de revêtement comprenant i°) au moins un composé polysaccharidique, ii°) au moins un collagène, et iii°) au moins un mélange de glycérol et de polyéthylène glycol,
- b°) on immerge la partie de la surface du substrat à revêtir dans la composition de revêtement obtenue en a°),
- c°) on retire ledit substrat de la composition de revêtement et on laisse sécher.

23. Procédé selon la revendication 22, **caractérisé en ce qu'**on prépare la composition de revêtement en mélangeant de façon homogène une solution de composé polysaccharidique, une solution de collagène, ainsi que le glycérol et le polyéthylène glycol.

24. Procédé selon la revendication 23, **caractérisé en ce que** la solution de composé polysaccharidique comprend, à l'état liquide, entre 7,5 et 15% en poids du composé polysaccharidique en solution aqueuse.

25. Procédé selon l'une quelconque des revendications 22 à 24, **caractérisé en ce que** la composition de revêtement présente une viscosité allant de 25 10⁻³ à 0,1 Pa.s.

26. Procédé selon l'une quelconque des revendications 22 à 25, **caractérisé en ce qu'**on immerge la partie de la surface du substrat métallique à revêtir dans la composition de revêtement afin de réaliser une enduction.

27. Procédé selon la revendication 26, **caractérisé en ce que** l'enduction a lieu à une température comprise entre 30 °C et 50°C, à un pH compris entre 5 et 8, durant un temps compris entre 2 secondes et 5 min.

28. Procédé selon la revendication 27, **caractérisé en ce que** l'immersion de la surface du substrat dans la composition de revêtement est de préférence réitérée à un nombre de reprises allant de 1 à 10.

29. Procédé selon l'une quelconque des revendications 22 à 28, **caractérisé en ce que** le séchage se fait sous rotation constante afin d'obtenir une couche ou un film de revêtement homogène sur toute la surface revêtue dudit substrat métallique.

30. Procédé selon l'une quelconque des revendications 22 à 29, **caractérisé en ce que** la surface revêtue est mise à sécher sous hotte à une température comprise entre 10 °C et 35°C et durant une durée comprise entre 6 et 36 heures.

31. Procédé selon l'une quelconque des revendications 22 à 30, **caractérisé en ce qu'**il comprend en outre une étape de stérilisation du substrat revêtu.

32. Procédé selon la revendication 31, **caractérisé en ce que** la stérilisation se fait par traitement au gaz d'éthylène.

33. Objet à usage médical ou chirurgical, du type implant, par exemple une endoprothèse vasculaire pour angioplastie coronarienne transluminale percutanée, comprenant un substrat métallique dont au moins une partie de la surface est revêtue par une composition de revêtement telle que définie à l'une quelconque des revendications 1 à 21.

34. Endoprothèse comprenant un substrat métallique selon l'une quelconque des revendications 1 à 21.

35. Endoprothèse selon la revendication 34, **caractérisée en ce que** le substrat métallique est un alliage, par exemple un acier inoxydable, ou un superalliage, par exemple le phynox.

## Claims

1. A metal substrate, **characterized in that** it is coated with a coating composition comprising:
- at least one polysaccharide compound,
- at least one collagen, and
- at least one mixture of glycerol and polyethylene glycol.

2. The substrate according to claim 1, **characterized in that** the polysaccharide compound comprises a polymeric chain consisting of a multiplicity of oside units, further called a polyoside chain, said chain being either substituted or not.

3. The substrate according to claim 1 or 2, **characterized in that** the polysaccharide compound comprises at least one group selected from methylcarboxylates, carboxymethylbenzylamides, sulfonates, including carboxymethylsulfonates, sulfates and their mixtures.

4. The substrate according to any of the preceding claims, **characterized in that** the polysaccharide compound further comprises at least one group selected from chains including hydroxyl functions, amide functions, benzyl groups and/or mixtures thereof.

5. The substrate according to any of claims 2 to 4, **characterized in that** the polyoside chain of the polysaccharide compound is that of a polysaccharide selected from the group consisting of starch, glycogen, celluloses, dextrans, poly-α-1,3-glucans, pullulans, chitin, arabans, xylans, fucans, pectins, chondroitin sulfates, mucopolysaccharides and their derivatives.

6. The substrate according to claim 5, **characterized in that** the polyoside chain of the polysaccharide compound is that of a dextran.

7. The substrate according to claim 6, **characterized in that** the polysaccharide compound is a dextran having an average molar mass comprised between 10,000 and 1,000,000 g/mol, having for example a molar mass equal to 40,000, 70,000 or 460,000 g/mol.

8. The substrate according to claim 6 or 7, **characterized in that** the polysaccharide compound is selected from compounds of general formula DMCₐB_{b}Su_{c}S_{d} wherein:
D represents a polysaccharide chain, formed by linkages of α-D-glucopyranose units connected together through α(1-6) bonds,
MC represents methylcarboxylate groups,
B represents carboxymethylbenzylamide groups,
Su represents sulfate groups,
S represents sulfonate groups, and
a, b, c and d represent the substitution degree (sd), expressed relatively to the number of free hydroxyl functions in a glucoside unit of the polysaccharide chain D, on groups MC, B, Su and S respectively, a being equal to 0 or ≥ 0.2, b being equal to 0 or ≥ 0.1, c being equal to 0 or ≥ 0.1 and d being equal to 0 or ≤ 0.15, provided that when d=0, a and/or b are ≠ 0.

9. The substrate according to claim 8, **characterized in that** the polysaccharide compound is selected from the group consisting of:
- polysaccharide compounds fitting the general formula DMCₐB_{b}Su_{c}S_{d} wherein a ≥0.7; 0.15≤ b ≤ 0.3; 0 ≤ c ≤ 0.15 and d = 0 or ≤0.1, and the average molar mass by weight is comprised between 5,000 and 200,000 g/mol,
- polysaccharide compounds fitting the general formula DMCₐB_{b}Su_{c}S_{d} wherein 0.4 ≤ a ≤ 0.8; 0.3 ≤ b ≤ 0.8; 0.1 ≤ c ≤0.9 and d = 0 or ≤ 0.1, and the average molar mass by weight of which is comprised between 5,000 and 200,000 g/mol,
- polysaccharide compounds fitting the general formula DMCₐB_{b}Su_{c}S_{d} wherein a ≥ 0.5; 0.3 ≤ b ≤ 0.5; c = 0 or ≤ 0.1 and d = 0 or ≤ 0.1, and the average molar mass by weight of which is comprised between 5,000 and 200,000 g/mol,
- and mixtures thereof.

10. The substrate according to claim 1, **characterized in that** the polysaccharide compound is naturally a bearer of sulfonate and/or sulfate groups.

11. The substrate according to claim 10, **characterized in that** the polysaccharide compound is selected from heparin, fucans, and mixtures thereof.

12. The substrate according to any of the preceding claims, **characterized in that** the collagen is selected from collagen of animal origin, collagen of human origin, such as for example human placenta collagen, collagen obtained by genetic recombination means and mixtures thereof.

13. The substrate according to claim 12, **characterized in that** the collagen is collagen of animal origin.

14. The substrate according to claim 12, **characterized in that** the collagen of animal origin is bovine or porcine collagen of type I.

15. The substrate according to any of claims 12 to 14, **characterized in that** the collagen is native collagen, solubilized at an acid pH or after treatment by digestion with pepsin.

16. The substrate according to any of claims 12 to 14, **characterized in that** the collagen is collagen modified by oxidative cutting.

17. The substrate according to any of claims 1 to 13, **characterized in that** the collagen is collagen of type I, extracted from animal dermises, by solubilization at an acid pH or by digestion with pepsin and purified by saline precipitations.

18. The substrate according to any of claims 1 to 17, **characterized in that** the polysaccharide compound is present in the coating composition at a content ranging from 0.01 % to 20% by weight, still preferably from 0.1 % to 10% by weight, based on the total weight of the coating composition.

19. The substrate according to any of claims 1 to 18, **characterized in that** the collagen is present in the coating composition at a content ranging from 0.01% to 10% by weight, preferably from 0.1 % to 5% by weight, based on the total weight of the coating composition.

20. The substrate according to any of claims 1 to 19, **characterized in that** the glycerol is present in the coating composition at a content ranging from 0.1 % to 5% by volume, still preferably from 0.3% to 3% by volume, based on the total volume of the coating composition.

21. The substrate according to any of claims 1 to 20, **characterized in that** the polyethylene glycol is present in the coating composition at a content ranging from 0.1 % to 5% by weight, still preferably from 0.3% to 3% by weight, based on the total weight of the coating composition.

22. A method for coating at least one portion of the surface of a metal substrate, **characterized in that** it comprises the following steps:
- a°) a coating composition is prepared, comprising i°) at least one polysaccharide compound, ii°) at least one collagen, and iii°) at least one mixture of glycerol and polyethylene glycol,
- b°) the portion of the substrate to be coated is immersed in the coating composition obtained in a°),
- c°) said substrate of the coating composition is removed and is left to dry.

23. The method according to claim 22, **characterized in that** the coating composition is prepared by homogeneously mixing a solution of polysaccharide compound, a solution of collagen, as well as the glycerol and the polyethylene glycol.

24. The method according to claim 23, **characterized in that** the solution of polysaccharide compound comprises in the liquid state between 7.5 and 15% by weight of the polysaccharide compound in an aqueous solution.

25. The method according to any of claims 22 to 24, **characterized in that** the coating composition has a viscosity ranging from 25.10⁻³ to 0.1 Pa.s.

26. The method according to any of claims 22 to 25, **characterized in that** the portion of the surface of the metal substrate to be coated is immersed in the coating composition in order to achieve a coating.

27. The method according to claim 26, **characterized in that** the coating takes place at a temperature comprised between 30°C and 50°C, at a pH comprised between 5 and 8, during a period of time comprised between 2 seconds and 5 mins.

28. The method according to claim 27, **characterized in that** the immersion of the surface of the substrate in the coating composition is preferably repeated for a number of times ranging from 1 to 10.

29. The method according to any of claims 22 to 28, **characterized in that** drying is accomplished under constant rotation in order to obtain a homogeneous coating layer or film on the whole coated surface of said metal substrate.

30. The method according to any of claims 22 to 29, **characterized in that** the coated surface is set to dry under a hood at a temperature comprised between 10°C and 35°C and for a period of time comprised between 6 and 36 hours.

31. The method according to any of claims 22 to 30, **characterized in that** it further comprises a step for sterilizing the coated substrate.

32. The method according to claim 31, **characterized in that** the sterilization is accomplished by treatment with ethylene gas.

33. An object for medical or surgical use of the implant type, for example a vascular endoprosthesis for percutaneous transluminal coronary angioplasty comprising a metal substrate, at least one portion of the surface of which is coated with a coating composition as defined according to any of claims 1 to 21.

34. An endoprosthesis comprising a metal substrate according to any of claims 1 to 21.

35. The endoprosthesis according to claim 34, **characterized in that** the metal substrate is an alloy, for example stainless steel, or a superalloy, for example phynox.

## Patentansprüche

1. Metallisches Substrat, **dadurch gekennzeichnet, dass** es mit einer Beschichtungszusammensetzung beschichtet ist, die umfasst:
- mindestens eine Polysaccharidverbindung,
- mindestens ein Kollagen und
- mindestens ein Gemisch aus Glycerol und Polyethylenglycol.

2. Substrat nach Anspruch 1, **dadurch gekennzeichnet, dass** die Polysaccharidverbindung eine Polymerkette umfasst, die von einer Vielfalt osidischer Motive gebildet wird, auch als Polyosidkette bezeichnet, wobei die Kette substituiert wird oder nicht.

3. Substrat nach Anspruch 1 oder 2, **dadurch gekennzeichnet, dass** die Polysaccharidverbindung mindestens eine Gruppe umfasst, die aus den Methylcarboxylaten, den Carboxymethylbenzylamiden, den Sulfonaten, darunter den Carboxymethylsulfonaten, den Sulfaten und ihren Gemischen ausgewählt ist.

4. Substrat nach einem der vorangehenden Ansprüche, **dadurch gekennzeichnet, dass** die Polysaccharidverbindung weiterhin mindestens eine Gruppe umfasst, die aus den Ketten ausgewählt ist, die Hyroxylfunktionen, Amdifunktionen, Benzylgruppen und/oder ihre Gemische enthalten.

5. Substrat nach einem der Ansprüche 2 bis 4, **dadurch gekennzeichnet, dass** die Polyosidkette der Polysaccharidverbindung die eines Polysaccharids ist, das aus der Gruppe ausgewählt ist, die von der Stärke, dem Glykogen, den Zellulosen, den Dextranen, den Poly-α-1,3-glucanen, den Pullulanen, dem Chitin, den Arabanen, den Xylanen, den Fucanen, den Pektinen, den Chondroitinsulfaten, den Mucopolysacchariden und ihren Derivaten gebildet wird.

6. Substrat nach Anspruch 5, **dadurch gekennzeichnet, dass** die Polyosidkette der Polysaccharidverbindung die eines Dextrans ist.

7. Substrat nach Anspruch 6, **dadurch gekennzeichnet, dass** die Polysaccharidverbindung ein Dextran ist mit einer durchschnittlichen molaren Masse zwischen 10000 und 1000000 g/mol inklusive mit beispielsweise einer molaren Masse von gleich 40000, 70000 oder 460000 g/mol.

8. Substrat nach Anspruch 6 oder 7, **dadurch gekennzeichnet, dass** die Polysaccharidverbindung aus den Verbindungen mit der allgemeinen Formel DMCₐB_{b}Su_{c}S_{d} ausgewählt ist, in der:
D eine Polysaccharidkette darstellt, die von Verkettungen von α-D-Glucopyranoseeinheiten gebildet wird, die untereinander durch α(1-6)-Bindungen verbunden sind,
MC Methylcarboxylatgruppen darstellt,
B Carboxymethylbenzylamidgruppen darstellt,
Su Sulfatgruppen darstellt,
S Sulfonatgruppen darstellt und
a, b, c und d den Substitutionsgrad (ds) darstellen, ausgedrückt im Verhältnis zur Anzahl freier Hydroxylfunktionen in einer glucosidischen Einheit der Polysaccharidkette D, jeweils in MC-, B-, Su- und S-Gruppen, wobei a gleich 0 oder ≥0,2, b gleich 0 oder ≥0,1, c gleich 0 oder ≥0,1 und d gleich 0 oder ≤ 0,15, wenn d = 0, wobei a und/oder b ≠ 0.

9. Substrat nach Anspruch 8, **dadurch gekennzeichnet, dass** die Polysaccharidverbindung aus der Gruppe ausgewählt ist, die gebildet wird von:
- den Polysaccharidverbindungen, die der allgemeinen Formel DMCₐB_{b}Su_{c}S_{d} entsprechen, in der a ≥ 0,7, 0,15 ≤ b ≤ 0,3, 0 ≤ c ≤ 0,15 und d = 0 oder ≤ 0,1 ist und deren durchschnittliche molare Gewichtsmasse zwischen 5000 und 200000 g/mol inklusive beträgt,
- den Polysaccharidverbindungen, die der allgemeinen Formel DMCₐB_{b}Su_{c}S_{d} entsprechen, in der 0,4 ≤ a ≤ 0,8, 0,3 ≤ b ≤ 0,8, 0,1 ≤ c ≤ 0,9 und d = 0 oder ≤ 0,1 ist und deren durchschnittliche molare Gewichtsmasse zwischen 5000 und 200000 g/mol inklusive beträgt,
- den Polysaccharidverbindungen, die der allgemeinen Formel DMCₐB_{b}Su_{c}S_{d} entsprechen, in der a ≥ 0,5, 0,3 ≤ b ≤ 0,5, c = 0 oder ≤ 0,1 und d = 0 oder ≤ 0,1 ist und deren durchschnittliche molare Gewichtsmasse zwischen 5000 und 200000 g/mol inklusive beträgt,
- und ihren Gemischen.

10. Substrat nach Anspruch 1, **dadurch gekennzeichnet, dass** die Polysaccharidverbindung natürlicher Träger von Sulfonat- und/oder Sulfatgruppen ist.

11. Substrat nach Anspruch 10, **dadurch gekennzeichnet, dass** die Polysaccharidverbindung aus dem Heparin, den Fucanen und ihren Gemischen ausgewählt ist.

12. Substrat nach einem der vorangehenden Ansprüche, **dadurch gekennzeichnet, dass** das Kollagen aus dem tierischen Kollagen, dem menschlichen Kollagen, wie zum Beispiel dem menschlichen Plazentakollagen, dem Kollagen, das durch Mittel der genetischen Rekombination gewonnen wurde, und ihren Gemischen ausgewählt ist.

13. Substrat nach Anspruch 12, **dadurch gekennzeichnet, dass** das Kollagen tierisches Kollagen ist.

14. Substrat nach Anspruch 12, **dadurch gekennzeichnet, dass** das tierische Kollagen Rinder- oder Schweinekollagen des Typs 1 ist.

15. Substrat nach einem der Ansprüche 12 bis 14, **dadurch gekennzeichnet, dass** das Kollagen natives Kollagen ist, das bei saurem pH oder nach Verarbeitung durch Verdauung mit Pepsin solubilisiert wurde.

16. Substrat nach einem der Ansprüche 12 bis 14, **dadurch gekennzeichnet, dass** das Kollagen durch oxidative Spaltung modifiziertes Kollagen ist.

17. Substrat nach einem der Ansprüche 1 bis 13, **dadurch gekennzeichnet, dass** das Kollagen Kollagen vom Typ 1 ist, das durch Solubilisierung bei sauren pH oder durch Verdauung mit Pepsin aus der Dermis von Tieren extrahiert und durch Aussalzen gereinigt wurde.

18. Substrat nach einem der Ansprüche 1 bis 17, **dadurch gekennzeichnet, dass** die Polysaccharidverbindung in der Beschichtungszusammensetzung in einem Gehalt vorhanden ist, der von 0,01 bis 20 Gew.-% reicht, vorzugsweise noch von 0,1 bis 10 Gew.-%, im Verhältnis zum Gesamtgewicht der Beschichtungszusammensetzung.

19. Substrat nach einem der Ansprüche 1 bis 18, **dadurch gekennzeichnet, dass** das Kollagen in der Beschichtungszusammensetzung in einem Gehalt vorhanden ist, der von 0,01 bis 10 Gew.-% reicht, vorzugsweise noch von 0,1 bis 5 Gew.-%, im Verhältnis zum Gesamtgewicht der Beschichtungszusammensetzung.

20. Substrat nach einem der Ansprüche 1 bis 19, **dadurch gekennzeichnet, dass** das Glycerol in der Beschichtungszusammensetzung in einem Gehalt vorhanden ist, der von 0,1 bis 5 Vol.-% reicht, vorzugsweise noch von 0,3 bis 3 Vol.-%, im Verhältnis zum Gesamtvolumen der Beschichtungszusammensetzung.

21. Substrat nach einem der Ansprüche 1 bis 20, **dadurch gekennzeichnet, dass** das Polyethylenglycol in der Beschichtungszusammensetzung in einem Gehalt vorhanden ist, der von 0,1 bis 5 Gew.-% reicht, vorzugsweise noch von 0,3 bis 3 Gew.-%, im Verhältnis zum Gesamtgewicht der Beschichtungszusammensetzung.

22. Verfahren zur Beschichtung mindestens eines Teils der Oberfläche eines metallischen Substrats, **dadurch gekennzeichnet, dass** es die folgenden Schritte umfasst:
- a) Vorbereitung einer Beschichtungszusammensetzung, die i) mindestens eine Polysaccharidverbindung, ii) mindestens ein Kollagen und iii) mindestens ein Glycerol- und Polyethylenglycerolgemisch umfasst,
- b) Eintauchen des zu beschichtenden Teils der Substratoberfläche in die in a) hergestellte Beschichtungszusammensetzung,
- c) Herausnehmen des Substrats aus der Beschichtungszusammensetzung und Trocknung.

23. Verfahren nach Anspruch 22, **dadurch gekennzeichnet, dass** die Beschichtungszusammensetzung durch homogenes Mischen einer Polysaccharidverbindungslösung, einer Kollagenlösung sowie des Glycerols und des Polyethylenglycols zubereitet wird.

24. Verfahren nach Anspruch 23, **dadurch gekennzeichnet, dass** die Lösung der Polysaccharidverbindung im flüssigen Zustand zwischen 7,5 und 15 Gew.-% inklusive der Polysaccharidverbindung in wässriger Lösung umfasst.

25. Verfahren nach einem der Ansprüche 22 bis 24, **dadurch gekennzeichnet, dass** die Beschichtungszusammensetzung eine Viskosität von 25 10⁻³ bis 0,1 Pa.s aufweist.

26. Verfahren nach einem der Ansprüche 22 bis 25, **dadurch gekennzeichnet, dass** der zu beschichtende Teil der Oberfläche des metallischen Substrats in die Beschichtungszusammensetzung eingetaucht wird, um eine Beschichtung durchzuführen.

27. Verfahren nach Anspruch 26, **dadurch gekennzeichnet, dass** die Beschichtung bei einer Temperatur zwischen 30 °C und 50 °C inklusive, bei einem pH zwischen 5 und 8 inklusive während einer Zeit zwischen 2 Sekunden und 5 Minuten inklusive stattfindet.

28. Verfahren nach Anspruch 27, **dadurch gekennzeichnet, dass** das Eintauchen der Oberfläche des Substrats in die Beschichtungszusammensetzung vorzugsweise 1 bis 10 Mal wiederholt wird.

29. Verfahren nach einem der Ansprüche 22 bis 28, **dadurch gekennzeichnet, dass** die Trocknung bei konstanter Rotation erfolgt, um eine homogene Beschichtungsschicht oder -film auf der gesamten Oberfläche, die mit dem metallischen Substrat beschichtet ist, zu erhalten.

30. Verfahren nach einem der Ansprüche 22 bis 29, **dadurch gekennzeichnet, dass** die beschichtete Oberfläche unter einem Abzug bei einer Temperatur zwischen 10 °C und 35 °C inklusive getrocknet wird bei einer Dauer zwischen 6 und 36 Stunden inklusive.

31. Verfahren nach einem der Ansprüche 22 bis 30, **dadurch gekennzeichnet, dass** es weiterhin einen Schritt der Sterilisierung des beschichteten Substrats umfasst.

32. Verfahren nach Anspruch 31, **dadurch gekennzeichnet, dass** die Sterilisierung durch Behandlung mit Ethylengas erfolgt.

33. Gegenstand zur medizinischen oder chirurgischen Verwendung vom Typ Implantat, zum Beispiel eine vaskuläre Endoprothese für perkutane transluminale Koronarangioplastie, der ein metallisches Substrat umfasst, von dem mindestens ein Teil der Oberfläche mit einer Beschichtungszusammensetzung beschichtet ist, wie in einem der Ansprüche 1 bis 21 definiert.

34. Endoprothese, die ein metallisches Substrat nach einem der Ansprüche 1 bis 21 umfasst.

35. Endoprothese nach Anspruch 34, **dadurch gekennzeichnet, dass** das metallische Substrat eine Legierung ist, zum Beispiel ein Edelstahl oder eine Superlegierung, zum Beispiel Phynox.
